# EUROPÄISCHE PATENTSCHRIFT

(11) **EP 1 321 431 B1**
(45) Veröffentlichungstag und Bekanntmachung des Hinweises auf die Patenterteilung: **19.09.2007**
(21) Anmeldenummer: 02027901.4
(22) Anmeldetag: 13.12.2002
(51) Int. Cl.: C01F 7/56, A61Q 15/00

(54) **Verwendung von Aluminiumchlorohydrat in Deodoranten und Antiperspiranten**
The use of aluminium chlorohydrate in deodorants and antiperspirants
L'utilisation du chlorohydrate d'aluminium dans déodorants et agents anti-transpiration

(30) Priorität: 21.12.2001 DE 10163242
(43) Veröffentlichungstag der Anmeldung: 25.06.2003
(73) Patentinhaber: Clariant Produkte (Deutschland) GmbH, 65929 Frankfurt am Main (DE)
(72) Erfinder: Klug, Peter, Dr., 63762 Grossostheim (DE); Rösch, Norbert, Dr., 86386 Gersthofen (DE); Reiser, Karl Werner, 30823 Garbsen (DE)

(56) Entgegenhaltungen:
- EP-A- 0 530 598
- WO-A-00/71091
- US-A- 5 908 616

## Beschreibung

Die vorliegende Erfindung betrifft die Verwendung von Aluminiumchlorohydrat mit einem Eisengehalt kleiner 20 ppm, bezogen auf 100 % Feststoff zur Verbesserung der Wirkung von Deodorantien und Antiperspirant-Produkten.

Der Einsatz basischer Aluminiumchloride in Deodorantien und Antiperspirantien zur Reduzierung von Schweißbildung ist seit langem bekannt.
Solche basischen Aluminiumchloride können, wie in DE-PS 23 09 610 beschrieben, durch Umsetzung von Aluminiumhydroxid mit Salzsäure bei Temperaturen zwischen 70°C und 140°C oder durch eine Redoxreaktion von Aluminium und Aluminiumchloridhydrat bei Temperaturen um 90 bis 100°C hergestellt werden.

Die makromolekulare Struktur der Aluminiumchlorohydrate ist sehr komplex und setzt sich aus unterschiedlichen Clusterpolyethern mit unterschiedlichen Molekulargewichten zusammen. Bekannt ist, dass die adstringierende Wirkung von Aluminiumchlorohydraten mit höherem Anteil an Clustern mit kleineren Molekulargewichten stärker ausgeprägt ist im Vergleich zu hochmolekularen Clusteranteilen. Diese sogenannten aktivierten Aluminiumchlorohydrate sind metastabil und verändern ihre Makrostruktur in Abhängigkeit von Temperatur, Konzentration der Lösung und Lagerungsdauer.

Die Verwendung von Aluminiumchlorohydraten als Antiperspirantwirkstoff in kosmetischen Formulierungen ist lange bekannt und gut dokumentiert. Die für kosmetische Anwendungen geeigneten Aluminiumchlorohydrate können, je nach eingesetzten Rohstoffen, Eisen und weitere Spurenmetalle in Mengen von 40 ppm bis 200 ppm enthalten, bezogen auf 100 % Trockensubstanz. Typische Werte liegen bei den heute verwendeten Aluminiumchlorohydraten bei ca. 40-100 ppm, bezogen auf 100 % Trockensubstanz. Dieser Eisengehalt rührt im wesentlichen aus dem Eisengehalt des zugrundeliegenden Aluminiumhydroxids bzw. Aluminiummetalls her. Als Handelsformen für Aluminiumchlorohydrate wird im allgemeinen eine 50 % wässrige Lösung, aber auch Schuppen, Sprühpulver und gemahlene Pulver verwendet.

Überraschend wurde nun gefunden, dass Aluminiumchlorohydrate eine signifikant verbesserte Wirkung in Antiperspirantformulierungen haben, wenn diese einen Eisengehalt unter 20 ppm, bevorzugt unter 10 ppm (bezogen auf 100 % Trockensubstanz) aufweisen. Dieser Effekt ist erstaunlich, da erwartet wurde, dass ein Spurenelement keinen entscheidenden Effekt auf die Wirkung der Aluminiumchlorohydrate zeigen sollte.

Gegenstand der Erfindung ist die Verwendung von Aluminiumchlorohydraten gemäß der Formel I

Al₂(OH)ₙCl_{z} (I)

wobei n eine Zahl zwischen 4,5 und 5,1 und z eine Zahl zwischen 1,5 und 0,9 ist und n + z stets 6 beträgt, mit einem Eisengehalt kleiner 20 ppm, bevorzugt kleiner 10 ppm, bezogen auf die Trockensubstanz, zur Verbesserung der Wirkung von Deodorantien und Antiperspirantien hinsichtlich der Schweißreduktion und der Verringerung der Intensität der Geruchsbildung.

Die erfindungsgemäß verwendeten hochreinen Aluminiumchlorohydrate werden erhalten, indem man wässrige Lösungen solcher Aluminiumchlorohydrate, die einen erhöhten Anteil von Eisen und anderen Metallen enthalten, bei Temperaturen von 70 bis 140°C mit metallischem Aluminium, beispielsweise in Form von Barren, Chips oder Granulat, in Kontakt bringt.

Der Gewichtsanteil der Aluminiumchlorohydrate in den Antiperspirantien beträgt typischerweise 0,01 Gew.-% bis 20 Gew.-%, bevorzugt 0,05 Gew.-% bis 15 Gew.-%, insbesondere bevorzugt 0,1 bis 10 Gew.-%, bezogen auf das fertige Mittel und 100 % Aktivsubstanz Aluminiumchlorohydrat.

Die Antiperspirantien können in unterschiedlichen Ausführungsformen wie Gele, Sticks, Cremes, Sprays, Puder, Pudersprays dargeboten werden und können alle üblichen Bestandteile wie weitere Adstringentien, antimikrobielle Wirkstoffe, Gelierungsmittel, Alkohole, Öle, Wachse, Emulgatoren und Co-Emulgatoren, Überfettungsmittel, feuchtigkeitsspendende Mittel, Stabilisatoren, biogene Wirkstoffe (Lokalanästhetika, Antibiotika, Antiphlogistik, Antiallergica, Corticosteroide, Sebostatika), Vitamine, Panthenol, Allantoin, Pflanzenextrakte, beispielsweise Aloe Vera und Proteine, Glycerin, Konservierungsmittel, Perlglanzmittel, Farb- und Duftstoffe, Lösungsmittel, Hydrotrope, Enzyme, Trägersubstanzen, beispielsweise Schichtsilikate, pyrogene Kieselsäure, Elektrolytsalze wie KCI, NaCI, Komplexbildner, Antioxidantien und UV-Lichtschutzfilter enthalten.

An weiteren Adstringentien in Betracht kommen Oxide, bevorzugt Magnesiumoxid, Aluminiumoxid, Titandioxid, Zirkondioxid und Zinkoxid, Oxidhydrate, bevorzugt Aluminiumoxidhydrat (Böhmit) und Hydroxide, bevorzugt von Calcium, Magnesium, Aluminium, Titan, Zirkon oder Zink.

Vorteilhafterweise enthalten die Antiperspirantien zusätzlich zu den Aluminiumchlorohydraten antimikrobielle Wirkstoffe, die die schweißzersetzenden Mikroorganismen bzw. das schweißzersetzende Esterase-Enzym hemmen. Bevorzugt geeignet als antimikrobielle Wirkstoffe sind Cetyltrimethylammoniumchlorid, Cetylpyridiniumchlorid, Benzethoniumchlorid, Diisobutylethoxyethyldimethylbenzylammoniumchlorid, Natrium N-Laurylsarcosinat, Natrium-N-Palmethylsarcosinat, Lauroylsarcosin, N-Myristoylglycin, Kalium N-Laurylsarcosin, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-trichlorocarbanilid (Triclocarban), Diaminoalkylamid, beispielsweise L-Lysinhexadecylamid, Citratschwermetallsalze, Salicylate, Piroctone, insbesondere Zinksalze, Pyrithione und deren Schwermetallsalze, insbesondere Zinkpyrithion, Zinkphenolsulfat, Farnesol und Kombinationen dieser Wirksubstanzen.

Die Mittel enthalten die antimikrobiellen Mittel bevorzugt in Mengen bis 50 Gew.-%, besonders bevorzugt 0,01 bis 10 Gew.-%, insbesondere bevorzugt 0,1 bis 5 Gew.-%.

Weiterhin bevorzugt enthalten die Mittel Gelierungsmittel.
Als Gelierungsmittel eignen sich alle oberflächenaktiven Stoffe, die in der flüssigen Phase gelöst eine Netzwerkstruktur ausbilden und so die flüssige Phase verfestigen. Geeignete Geliermittel sind z.B. in WO 98/58625 genannt.
Bevorzugte Gelierungsmittel sind Metallsalze von Fettsäuren, bevorzugt mit 12 bis 22 C-Atomen, beispielsweise Natriumstearat, Natriumpalmitat, Natriumlaurat, Natriumarachidate, Natriumbehenat, Kaliumstearat, Kaliumpalmitat, Natriummyristat, Kaliumpalmitat, Aluminiummonostearat, Hydroxyfettsäuren, beispielsweise 12-Hydroxystearinsäure, -lauryl-, 16-Hydroxyhexadecansäure; Fettsäureamide; Fettsäurealkanolamide; Dibenzalsorbit und alkohollösliche Polyamide und Polyacrylamide oder Mischungen solcher.
Weiterhin können die Mittel auch Gelierungsmittel in Form von Alkylsilikonwachsen enthalten. Geeignete Wachse sind z.B. C₂₀-₂₄-Alkyldimethicone (SilCare Silicone 41 M70), C₂₄-C₂₈-Alkyldimethicone (SilCare Silicone 41 M80), C₂₀₋₂₄ Alkylmethicone (SilCare Silicone 41 M40), C₂₄₋₂₈-Alkylmethicone (SilCare Silicone 41 M50) und C₃₀₋₄₅ Alkylmethicone.
Bevorzugt enthalten die Mittel 0,01 bis 20 Gew.-%, besonders bevorzugt 0,1 bis 10 Gew.-%, insbesondere bevorzugt 1 bis 8 Gew.-% und ganz besonders bevorzugt 3 bis 7 Gew.-% an Geliermitteln.

In einer weiteren bevorzugten Ausführungsform enthalten die Mittel zusätzliche Alkohole. Bevorzugte Alkohole sind alkoxylierte Alkohole mit bevorzugt 1 bis 80, besonders bevorzugt 3 bis 20, Alkoxygruppen und mindestens einer freien Hydroxylgruppe.
Besonders bevorzugt sind Ethanol, Propanol, Isopropanol, n-Butanol, i-Butanol, t-Butanol, Glycerin, Diethylenglykol, Triethylenglycol, Dipropylenglycol, Tripropylenglycol, flüssige Polypropylenpolyethylenglycol-Copolymere, Tetrapropylenglycol, Tetraethylenglycol, Dibutylenglycol, Trimethylenglycol, Diethylenglycolmonoethylether, PEG-8, 1,3-Butandiol, 1,4-Butandiol, Glycerolpropoxylat, Propylenglykol, Hexylenglykol, 1,2-Hexandiol, 1,3-Butylenglycol, 1,2,3-Trihydroxyhexan und 1,2,3-Trihydroxhexan. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45 Gew.-% und von Polyethylenglykol mit einer relativen Molekülmasse zwischen 400 und 600 in Mengen von 5 bis 25 Gew.-% bevorzugt. Die Mittel enthalten bevorzugt 5 bis 90 Gew.-%, besonders bevorzugt 5 bis 80 Gew.-% und insbesondere bevorzugt 20 bis 60 Gew.-% an Alkohol.

Als Duft- bzw. Parfümöle können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethyl-methylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 C-Atomen, Citral, Citronellal, Citronellyloxyacetaldehyd, Cycllamenaldehyd, Hydroxycitronellal, Lilial und Bourgeonal, zu den Ketonen z.B. die lonone, alpha-Isomethylionon und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.
Parfümöle können auch natürliche Riechstoffgemische enthalten, wie sie aus pflanzlichen oder tierischen Quellen zugänglich sind, z.B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl. Die Mittel können als weitere Hilfs- und Zusatzstoffe Ölkörper, Emulgatoren und Co-Emulgatoren, Überfettungsmittel, feuchtigkeitsspendende Mittel, Stabilisatoren, biogene Wirkstoffe (Lokalanästhetika, Antibiotika, Antiphlogistik, Antiallergica, Corticosteroide, Sebostatika), Vitamine, Panthenol, Allantoin, Pflanzenextrakte, beispielsweise Aloe Vera und Proteine, Glycerin, Konservierungsmittel, Perlglanzmittel, Farb- und Duftstoffe, Lösungsmittel, Hydrotrope, Enzyme, Trägersubstanzen, beispielsweise Schichtsilikate, pyrogene Kieselsäure, Elektrolytsalze wie KCI, NaCI, Komplexbildner, Antioxidantien und UV-Lichtschutzfilter enthalten.

Unter Ölkörper ist jegliche Fettsubstanz zu verstehen, die bei Raumtemperatur (25°C) flüssig ist.
Die Fett-Phase kann ein oder mehrere Öle umfassen, die vorzugsweise aus folgenden Ölen ausgewählt werden:
a) Silikonöle, flüchtig oder nicht flüchtig, linear, verzweigt oder ringförmig, eventuell organisch modifiziert, Phenylsilikone, Silikonharze und -gummis, die bei Raumtemperatur fest oder flüssig sind,
b) Mineralöle, wie Paraffin- oder Vaselinöl
c) Öle tierischen Ursprungs, wie Perhydrosqualen oder Lanolin;
d) Öle pflanzlichen Ursprungs, wie flüssige Triglyceride, z.B. Sonnenblumen-, Mais-, Soja-, Reis-, Jojoba-, Babusscu-, Kürbis-, Traubenkern-, Sesam-, Walnuss-, Aprikosen-, Makadamia-, Avocado-, Süßmandel-, Wiesenschaumkraut-, Ricinusöl, Triglyceride der Capryl/Caprinsäuren, Olivenöl, Erdnussöl, Rapsöl und Kokosnussöl;
e) synthetische Öle, wie Purcellinöl, Isoparaffine, lineare und/oder verzweigte Fettalkohole und Fettsäureester, bevorzugt Guerbetalkohole mit 6 bis 18, vorzugsweise 8 bis 10, Kohlenstoffatomen; Ester von linearen (C₆-C₁₃)-Fettsäuren mit linearen (C₆-C₂₀)-Fettalkoholen; Ester von verzweigten (C₆-C₁₃)-Carbonsäuren mit linearen (C₆-C₂₀)-Fettalkoholen, Ester von linearen (C₆-C₁₈)-Fettsäuren mit verzweigten Alkoholen, insbesondere 2-Ethylhexanol; Ester von linearen und/oder verzweigten Fettsäuren mit mehrwertigen Alkoholen (wie z.B. Dimerdiol oder Trimerdiol) und/oder Guerbetalkoholen; Triglyceride auf Basis (C₆-C₁₀)-Fettsäuren,
f) Ester, wie Dioctyladipate, Diisopropyl dimer dilinoleate, Propylenglycole/-dicaprilate oder Wachse wie Bienenwachs, Paraffinwachs oder Mikrowachse, gegebenenfalls in Kombination mit hydrophilen Wachsen, wie z.B. Cetylstearylalkohol,
g) fluorierte und perfluorierte Öle;
h) fluorierte Silikonöle;
i) Gemische der obengenannten Substanzen.

Als nichtionogene Co-Emulgatoren kommen u.a. in Betracht Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; (C₁₂-C₁₈)-Fettsäuremono- und -diester von Anlagerungsprodukten von 0 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und ggfs. deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z.B. Polyglycerinpolyricinoleat und Polyglycerinpoly-12-hydroxystearat. Ebenfalls geeignet sind Gemische von Verbindungen aus mehreren dieser Substanzklassen.

Als ionogene Co-Emulgatoren eignen sich z.B. anionische Emulgatoren, wie mono-, di- oder tri-Phosphorsäureester, aber auch kationische Emulgatoren wie mono-, di- und tri-Alkylquats und deren polymere Derivate.

Als Überfettungsmittel können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Als feuchtigkeitsspendende Substanz stehen beispielsweise Isopropylpalmitat, Glycerin und/oder Sorbitol zu Verfügung.

Als Stabilisatoren können Metallsalze von Fettsäuren, wie z.B. Magnesium-, Aluminium- und/oder Zinkstearat eingesetzt werden. Unter biogenen Wirkstoffen sind beispielsweise Pflanzenextrakte und Vitaminkomplexe zu verstehen.

Die Mittel können mit konventionellen Ceramiden, Pseudoceramiden, Fettsäure-N-alkylpolyhydroxyalkylamide, Cholesterin, Cholesterinfettsäureestern, Fettsäuren, Triglyceriden, Cerebrosiden, Phospholipiden und ähnlichen Stoffen als Pflegezusatz abgemischt werden.

Um die rheologischen Eigenschaften einzustellen, werden in der Fachliteratur eine Vielzahl von unterschiedlichen Systemen angegeben. Bekannt sind beispielsweise Celluloseether und andere Cellulosederivate (z.B. Carboxymethylcellulose, Hydroxyethylcellulose), Gelatine, Stärke und Stärkederivate, Natriumalginate, Fettsäurepolyethylenglykolester, Agar-Agar, Traganth oder Dextrine. Als synthetische Polymere kommen verschiedene Materialien zum Einsatz, wie z.B. Polyvinylalkohole, Polyacrylamide, Polyvinylamide, Polysulfonsäuren, Polyacrylsäure, Polyacrylsäureester, Polyvinylpyrrolidon, Polyvinylmethylether, Polyethylenoxide, Copolymere aus Maleinsäureanhydrid und Vinylmethylether, kammförmige, wahlweise gepfropften Acryloyldimethyltaurat enthaltende Copolymere, die in der Haupt- oder Seitenkette zudem kationische Ladungen bzw. Silizium-, Fluor oder Phosphoratome enthalten können, sowie diverse Mischungen und Copolymerisate aus den o.a. Verbindungen, einschließlich ihrer verschiedenen Salze und Ester. Diese Polymere können wahlweise vernetzt oder unvernetzt sein.

Als UV-Filter eignen sich z.B. 4-Aminobenzoesäure; 3-(4'-Trimethylammonium)benzyliden-boman-2-on-methylsulfat; 3,3,5-Trimethyl-cyclohexylsalicylat; 2-Hydroxy-4-methoxybenzophenon; 2-Phenylbenzimidazol-5-sulfonsäure und ihre Kalium-, Natrium- und Triethanolaminsalze; 3,3'-(1,4-Phenylendimethin)-bis-(7,7-dimethyl-2-oxobicyclo[2.2.1]-heptan-1-methansulfonsäure und ihre Salze; 1-(4-tert.-Butylphenyl)-3-(4-methoxyphenyl)propan-1,3-dion, 3-(4'-Sulfo)-benzyliden-boman-2-on und seine Salze; 2-Cyan-3,3-diphenyl-acrylsäure-(2-ethylhexylester); Polymer von N-[2(und 4)-(2-oxoborn-3-ylidenmethyl)benzyl]-acrylamid; 4-Methoxy-zimtsäure-2-ethyl-hexylester; ethoxyliertes Ethyl-4-amino-benzoat; 4-Methoxy-zimtsäureisoamylester; 2,4,6-Tris-[p-(2-ethylhexyloxycarbonyl)anilino]-1,3,5-triazin; 2-(2H-benzotriazol-2-yl)-4-methyl-6-(2-methyl-3-(1,3,3,3-tetramethyl-1-(trimethylsilyloxy)-disiloxanyl)propyl)phenol; 4,4'-[(6-[4-((1,1-dimethylethyl)-aminocarbonyl)phenylamino]-1,3,5-triazin-2,4-yl)diimino]bis-(benzoesäure-2-ethylhexylester); 3-(4'-Methylbenzyliden)-D,L-Campher; 3-Benzyliden-Campher; Salicylsäure-2-ethylhexylester; 4-Dimethylaminobenzoesäure-2-ethylhexylester; Hydroxy-4-methoxy-benzophenon-5-sulfonsäure (Sulisobenzonum) und das Natriumsalz und/oder 4-Isopropylbenzylsalicylat.

Als Antioxidantien stehen beispielsweise Superoxid-Dismutase, Tocopherol (Vitamin E) und Ascorbinsäure (Vitamin C) zur Verfügung. Als Konservierungsmittel in Betracht kommen beispielsweise Phenoxyethanol, Parabene, Pentandiol oder Sorbinsäure. Als Farbstoffe können die für kosmetische Zwecke geeigneten und zugelassenen Substanzen verwendet werden.

Bevorzugt handelt es sich bei den Deodorantien und Antiperspirantien um Lotionen, Cremes, Stifte (auch Mehrphasenstifte), Sprays, Roll-On-Präparate und Puder. Die Mittel haben einen pH-Wert von 2 bis 12, bevorzugt von 3-8, besonders bevorzugt von 3,5 bis 6.

Zur Bestimmung der Antiperspirantwirksamkeit wurden in einem Vergleichstest 50 %ige Aluminiumchlorohydratlösungen mit einem Resteisengehalt von ca. 40 ppm (Vergleichsbeispiel 1, Locron^{®} L, Clariant, 80 ppm Eisen auf 100 % Trockensubstanz) mit einer 50 % Aluminiumchlorohydratlösung mit einem Restgehalt von 10 ppm Eisen auf 100 % Trockensubstanz (Beispiel 1) verglichen.

Als Testformulierung wurde ein Antiperspirant Pumpspray der folgenden Rezeptur verwendet:

| | |
|---|---|
| A Wasser | 40 % |
| B Aluminiumchlorohydrat-Testprodukt (50 %ige Lösung in Wasser) | 20 % |
| Ethanol | 40 % |

Herstellvorschrift: Die Komponenten B zu A zugeben und homogen rühren.

### Beispiel 1:

Es wurde an 20 Probanden in der Achselhöhle folgender Antiperspirant-Test durchgeführt:

### Run-In-Phase:

Hierbei durften die Probanden sich 10 Tage nur mit einem milden Syndet ohne Parfümzusatz oder antibakteriellen Wirkstoff waschen und kein anderes Kosmetikum benutzen.

### Blindversuch:

Es wurde zunächst die Achselhöhle mit Zellstoff getrocknet, kleine Silicagelsäckchen angebracht und über 24 Stunden der abgesonderte Schweiß gravimetrisch durch Wägung der Silicagelsäckchen bestimmt.

### Vergleichsversuch:

Die Effektivität der beiden zu vergleichenden Antiperspirantwirkstoffes wurde dann mit dem entsprechenden Antiperspirant geprüft. Hierzu wurde das Pumpspray bis zur ablaufenden Nässe angewandt, über Nacht einwirken lassen, dann die Silicagelsäckchen angebracht und erneut über 24 Stunden das Schweißvolumen über die Gewichtszunahme der Silicagelsäckchen bestimmt.

Die Schweißreduktion (in %, bezogen auf den Blindversuch) nach einmaliger Anwendung war:
Vergleichsbeispiel 1 (80 ppm Resteisengehalt): 21 %
Beispiel 1 (10 ppm Resteisengehalt): 35 %

### Beispiel 2:

Es wurde an 20 Probanden jeweils an der Achselhöhle ein Antiperspirant-Test mit den obigen Formulierungen aus Beispiel 1 durchgeführt:
Es wurde die Intensität der Geruchsbildung nach Applikation der entsprechenden Antiperspirantformulierung geprüft (Snifftest):

### Run-In-Phase:

Hierzu wurden die Probanden folgendermaßen konditioniert:
Waschen über 3 Tage nur mit mildem Syndet ohne antibakteriellen Wirkstoff oder Parfüm. Während der Studie wurde nur das unparfümierte Syndet einmal täglich angewandt.

Geruchsbestimmungen wurden
a) vor der ersten Anwendung der Versuchsprodukte 24 h nach der letzten Waschung
b) nach 7-tägiger Anwendung der Antiperspirants, nach 8 und 24 Stunden nach Anwendung durchgeführt.

Die Geruchsbildung wurde von einem trainierten Expertenpanel (3 Personen) beurteilt.
Die Evaluierung erfolgte auf einer Skala von 0 = kein Geruch bis 6 = sehr starker Geruch.

### Startwert:

5,1 für beide Arme
nach 8 Stunden:

| | |
|---|---|
| Vergleichsbeispiel 1 (80 ppm Resteisengehalt): | 3,1 |
| Beispiel 1 (10 ppm Resteisengehalt): | 2,5 |

nach 24 Stunden:

| | |
|---|---|
| Vergleichsbeispiel 1 (80 ppm Resteisengehalt): | 4,1 |
| Beispiel 1 (10 ppm Resteisengehalt): | 3,2 |

Die Versuchsbeispiele zeigen, dass die Verwendung eisenarmer Locronqualitäten mit Eisengehalten < 20 ppm zu einer deutlichen und signifikanten Verbesserung der Antiperspirantwirkung führen.

Die nachfolgenden Beispiele beschreiben weitere Zubereitungen (bei allen Prozentangaben handelt es sich um Gew.-%).

### Beispiel 3: Antiperspirant-Creme

| | | |
|---|---|---|
| A | Cithrol GMS A/S | 15,0 % |
| | Glyceryl Stearat/PEG-100 Stearat | |
| | Crodacol C90 EP | 5,0 % |
| | Cetylalkohol | |
| | Aristoflex HMB | 2,0 % |
| | | |
| B | Wasser | ad 100 % |
| | Sorbit (70%ig) | 3,0 % |
| | Sorbitol | |
| | Aluminiumchlorohydrat aus Beispiel 1 | 44,5 % |
| | | |
| C | Parfümöl | q.s |
| | Farbstoffe | q.s |
| | Duftstoffe | q.s. |

### Herstellung:

I A und B ohne Reach 501 auf 70 bis 75°C erhitzen
II Unter Rühren abkühlen lassen
III Reach 501 bei 50 bis 55°C hinzufügen
IV Parfümöl bei 40 bis 45°C hinzugeben
V Bei 30 bis 35°C homogenisieren

### Beispiel 4: Klares Antiperspirant-Gel

| | | |
|---|---|---|
| A | Abil EM97 | 2,3 % |
| | Dimethicon Copolyol/Cyclopentasiloxan. | |
| | Abil B8839 | 6,9 % |
| | Cyclopentasiloxan/ Cyclohexasiloxan | |
| | Abil K4 | 6,9 % |
| | Cyclotetrasiloxan/ Cyclopentasiloxan | |
| | Aristoflex HMB | 1,5 % |
| B | Aluminiumchlorohydrat aus Beispiel 1 | 40,0 % |
| | Propylenglykol | 25,0 % |
| | Wasser dest. | ad 100 % |
| | Parfümöl | q.s. |

### Herstellung:

I Komponenten A und B separat mischen
II Phase B langsam unter Rühren bei Raumtemperatur zur Phase A geben.
III Homogenisieren

### Beispiel 5: W/O-Antiperspirant Creme

| | | |
|---|---|---|
| A | Abil EM90 | 2,0 % |
| | Cetyldimethicon/ Copolyol | |
| | Abil B8839 | 20,0 % |
| | Cyclopentasiloxan/ Cyclohexasiloxan | |
| | Aristoflex HMB | 2,0 % |
| B | Aluminium Chlorohydrat aus Beispiel 1 | 17,0 % |
| | Wasser dest. | ad 100 % |
| | Parfümöl | q.s. |
| | Konservierungsmittel | q.s. |

### Herstellung:

I Phase B langsam unter Rühren bei Raumtemperatur zur Phase A geben.
II Homogenisieren

### Beispiel 6: O/W-Antiperspirant-Creme

| | | |
|---|---|---|
| A | Aristoflex HMB | 2,0 % |
| | Arlamol ISML | 2,0 % |
| | Isosorbidlaurat | |
| | Dow Corning 245 Fluid | 2,0 % |
| | Cyclomethicon | |
| B | Wasser dest. | ad 100 % |
| | Aluminiumchlorohydrat aus Beispiel 1 | 40,0 % |
| | Konservierungsmittel | q.s |
| | Parfüm | q.s. |

### Herstellung

I Die Komponenten von A mischen
II Unter Rühren B zu A geben

- Aristoflex^{®} HMB:: Ammoniumacrylyldimethyltaurate behenet-25 methacrylat Copolymer

### Beispiel 7: Antiperspirant-Roll-On

| | | |
|---|---|---|
| A | Caprylyl Trimethicone | 0,30 % |
| | (SilCare^{®} Silicone 31 M 50) | |
| | Steareth-20 | 3,00 % |
| | (GENAPOL^{®} HS 200) | |
| | Steareth-2 | 1,50 % |
| | (GENAPOL^{®} HS 020) | |
| | Dicaprylyl Ether | 2,00 % |
| | (Cetiol^{®} OE) | |
| | Coco Caprylate/Caprate | 2,00 % |
| | (Cetiol^{®} LC) | |
| | Glycerin | 2,00 % |
| | Glyceryl Stearate | 2,00 % |
| | (Cutina^{®} GMS) | |
| | Octyldodecanol | 1,00 % |
| | (Eutanol^{®} G) | |
| | Stearyl alcohol | 2,50 % |
| B | Wasser | ad 100 % |
| | Aluminiumchlorohydrat nach Beispiel 1 | 10,00 % |
| | Avocado Extract | 0,30 % |
| | Persea Gratissima | |
| C | Parfüm | 0,30 % |

### Herstellung

| | |
|---|---|
| A | bei ca. 70°C aufschmelzen; |
| B | auf ca. 70°C erwärmen; |
| | Komponenten B zu Komponenten A geben und Rühren bis 35°C erreicht war; |
| | bei 35°C Komponente C zugeben. |

### Beispiel 8: Antiperspirant-Stick

| | | |
|---|---|---|
| A | Phenyl Trimethicone | 13,80 % |
| | (SilCare^{®} Silicone 15 M 50) | |
| | Cetearyl Alcohol | 19,30 % |
| | Cetiol CC | 13,80 % |
| | (Dicaprylyl Carbonate) | |
| | Stearinsäure | 3,50 % |
| | PEG-40 Hydrogenated Castor Oil | 4,10 % |
| | (Emulsogen^{®} HCO 040) | |
| | PEG-8 Distearate | 4,10 % |
| | (Cithrol 4 DS) | |
| | Petrolatum | 6,90 % |
| B | Aluminiumchlorohydrat nach Beispiel 1 | 34,50 % |
| | Aluminum Chlorohydrate | |

### Herstellung

| | |
|---|---|
| A | bei ca. 70°C aufschmelzen; |
| B | auf ca. 70°C erwärmen; |
| | Komponenten B unter Rühren zu Komponenten A geben; auf ca. 50°C abkühlen und abfüllen. |

## Patentansprüche

1. Verwendung von Aluminiumchlorohydraten gemäß der Formel I
Al₂(OH)ₙCl_{z} (I)
wobei n eine Zahl zwischen 4,5 und 5,1 und z eine Zahl zwischen 1,5 und 0,9 ist und n + z stets 6 beträgt, mit einem Eisengehalt kleiner 20 ppm, bevorzugt kleiner 10 ppm, bezogen auf die Trockensubstanz, zur Verbesserung der Wirkung von Deodorantien und Antiperspirantien hinsichtlich der Schweißreduktion und der Verringerung der Intensität der Geruchsbildung.

2. Verwendung nach Anspruch 1, **dadurch gekennzeichnet, dass** die Deodorantien und Antiperspirantien 0,01 bis 20 Gew.-% Aluminiumchlorohydrat enthalten.

3. Verwendung nach Anspruch 1, **dadurch gekennzeichnet, dass** die Deodorantien und Antiperspirantien als Gel, Stick, Creme, Spray, Puder oder Puderspray vorliegen.

## Claims

1. The use of aluminum chlorohydrates according to the formula I
Al₂(OH)ₙCl₂ (I)
where n is a number between 4.5 and 5.1 and z is a number between 1.5 and 0.9 and n + z is always 6, with an iron content of less than 20 ppm, preferably less than 10 ppm, based on the dry substance, for improving the action of deodorants and antiperspirants with regard to reducing perspiration and decreasing the intensity of odor formation.

2. The use as claimed in claim 1, wherein the deodorants and antiperspirants comprise 0.01 to 20% by weight of aluminum chlorohydrate.

3. The use as claimed in claim 1, wherein the deodorants and antiperspirants are in the form of a gel, stick, cream, spray, powder or powder spray.

## Revendications

1. Utilisation de chlorhydrates d'aluminium selon la formule I
Al₂(OH)ₙCl_{z} (I)
dans laquelle n est un nombre compris entre 4,5 et 5, 1 et z un nombre compris entre 1, 5 et 0,9 et n + z est toujours égal à 6, avec une teneur en fer inférieure à 20 ppm, de préférence inférieure à 10 ppm, par rapport à la substance sèche, pour améliorer l'action de déodorants et d'antitranspirants visant à diminuer la quantité de sueur et à réduire l'intensité de la formation d'odeurs.

2. Utilisation selon la revendication 1,
**caractérisée en ce que**
les déodorants et les antitranspirants contiennent de 0,01 à 20 % en poids de chlorhydrate d'aluminium.

3. Utilisation selon la revendication 1,
**caractérisée en ce que**
les déodorants et les antitranspirants se présentent sous forme de gel, de stick, de crème, de spray, de poudre ou de spray en poudre.
